# EUROPEAN PATENT APPLICATION

(11) **EP 1 864 671 A1**
(43) Date of publication of application: **12.12.2007**
(21) Application number: 06705725.7
(22) Date of filing: 06.03.2006
(51) Int. Cl.: A61K 33/20, A61P 9/12, A61P 1/16

(54) **THE USE OF HYDROCHLORIC ACID FOR THE MANUFACTURE OF A MEDICAMENT FOR THE TREATMENT OF HYPERTENSION**

(30) Priority: 10.03.2005 CN 200510049329
(71) Applicant: Liu, Zhiren, HHangzhou, Hzhejiang 310006 (CN)
(72) Inventor: Liu, Zhiren, HHangzhou, Hzhejiang 310006 (CN)
(74) Representative: Ellwanger, Arndt
(86) International application number: PCT/CN2006/000329
(87) International publication number: WO 2006/094451

(57) **Abstract**

The present invention relates to the use hydrochloric acid (HCl) for the manufacture of medicament for the treatment of hypertension as well as calculus, wherein, the medicament is particularly an aqueous solution that contains HCl and the pH of said aqueous solution is 3-6.7. The medicament which is prepared according to the invention can treat hypertension safely, and have the sure and evident effect, and can improve the arteriosclerosis of the hypertension patients, and can translate the calcium salts which are hazardous to human body to the calcium required by human body.

## Description

### I. Field of the Invention

The present invention relates to the use hydrochloric acid (HCl) for the manufacture of medicament for the treatment of hypertension as well as calculus.

### II. Background of the Invention

Many factors are responsible for human hypertension, including predisposition, endocrine disorder and bad diet. It is difficult to find a method to cure hypertension. Thus the medical community thinks hypertension is incurable requiring patients to take anti-hypertension drugs lifetime. Nowadays all drugs and instruments used to treat hypertension are intended to dilate arterial vessels, faster aging the vessels due to recurrent dilation. Even worse, dropping out of debris from wall of the vessels due to dilation of the vessels may cause myocardial infarct.

If hypertension is heritable, a newborn should be a hypertension patient, but this kind of newborn is hard to be found; if hypertension is caused by bad diet, no patient has been cured by changing his/her diet so far. These evidences defy the idea that patients suffering from hypertension should take anti-hypertension drugs lifetime. Meanwhile, if hypertension is caused by aged and hardened arterial vessels, merely usage of drugs or instruments is unable to dilate the vessels. Thus the idea that hypertension is caused by aged and hardened arterial vessels is untenable.

### III. Summary of the Invention

The present invention is to use HCl to the manufacture of medicament for the treatment of hypertension.

Inventors of the present invention after many years' endeavors find the mechanism of hypertension is as below: 1) Calcium bicarbonate in water and food can decompose forming binders such as calcium carbonate and calcium oxalate. The binders can attach human metabolites (such crystal of cholesterol) and other solid materials that enter into human body onto inner walls of arterial and capillary vessels. With age rising the attached materials onto the inner walls become thicker and thicker and aperture of the vessels become narrower and narrower, blocking blood flow and increasing blood pressure.

In addition, deposition of calcium carbonate and calcium oxalate in kidney, liver and gallbladder results in renal, hepatic and biliary calculus.
2) Materials attached to the inner walls of the vessels reduce flexibility of the vessels, baffling self-modulation of blood pressure;
3) Long attaching of the Materials to the inner walls of the vessels insidiously destroys blood vessels, reducing flexibility of the vessels.

Thus if the materials that attached to the inner walls of the vessels can be dissolved, blood pressure can restore normal, and to dissolve the attached materials the calcium carbonate and calcium oxalate should be dissolved, releasing and discharging other small particles. The inventors of the present invention find that HCl is the best material to dissolve calcium carbonate and calcium oxalate. Meanwhile, HCl can be absorbed to inner walls by protective membrane of the blood vessels, thus repairing the damaged vessels and restoring their original elasticity.

In addition, the inventors of the present invention find that HCl is able to help dissolve renal, hepatic and biliary calculus alleviating or removing calculus symptoms.

In accordance with one aspect of the invention there is provided a method that applying HCl to the manufacture of anti-hypertension medicines.

In another preferred embodiment, pH of the said aqueous solution is in the range of 3 to 6.9 (4-6.5 is preferable).

In another preferred embodiment, pH of the said aqueous solution is in the range of 6.5 to 6.9.

In another preferred embodiment, the said drug is in the form of oral formulation.

In another preferred embodiment, the said drug is in the form of injection formulation.

In another preferred embodiment, the said drug is an aqueous solution of HCl having a pH of 3-6.9 (4-6.5 is preferable), and the aqueous solution meets drinkable water requirements.

In another preferred embodiment, the said drug is an aqueous solution of HCl having a pH of 3-6.9, and the aqueous solution contains extra anti-hypertension medications that can keep stable within the above pH ranges.

According to another aspect of the invention there is provided a method to treat hypertension, i.e., administer the subjects with 100-3000ml of HCl aqueous solution of pH 3-6.9 each day for 0.5-6 years.

Preferably, the pH range of the aqueous solution is 4-6.5 or 6.5-6.9.

According to another aspect of the invention there is provided a kind of anti-hypertension formulation, which is an HCl aqueous solution of pH 3-6.9, and the aqueous solution contains extra anti-hypertension medications that can keep stable within the above pH ranges.

In another preferred embodiment, the extra anti-hypertension medications are selected from the group consisting but are not limited to Amlodipine, Benazepril, Captopril, Clonidine, Enalaprilat, Felodipin, Guanazodine, Indanidine, Indapamide, Minoxidil, Nitrendipine, Perindopril, Proroxan, Reserpiline, Trimoxamine, Tripamide, Utibapril, Zabiciprilat or their combinations.

Preferably, the said formulation is injection formulation.

According to yet another aspect of the invention, this invention provides HCl to the manufacture of medicines treating calculus.

In another preferred embodiment, the calculus diseases are selected from the group consisting renal calculus, hepatic calculus or biliary calculus.

The said drug in the present invention is ,usually an aqueous solution that contains HCl when this invention uses HCl to prepare medications to treat hypertension or calculus, and pH of the aqueous solution is in the range of 3 to 6.9 (4-6.5 is preferable). China's national standard requires drinkable water to have a pH of 6.5-8.5, indicating the aqueous solution that contains HCl can be directly drunk as edible HCl water if the above aqueous solution is adjusted to have pH between 6.5 and 6.9.

The said drug can be prepared for oral formulation, or injection formulation, or other formulation forms. Oral intake of HCl aqueous solution should be not less than 1500ml each day if it is used to treat hypertension or calculus, and in three to six divided doses a day and 3-6 months make up a therapeutic duration.. If the solution is injected for treating hypertension, recommended daily dosage is 500ml, two divided doses for in vein use, 2-3 months make up a therapeutic duration. If the above said method and dosage are adopted to treat hypertension, one year later the dosage can be reduced by a quarter, by a half two years later, and three years later, the patient merely needs a maintenance dosage or can be relieved for taking anti-hypertension medications but keep a normal blood pressure.

The HCl aqueous solution for oral use can be artificially prepared according to the following method: pour HCl into drinkable water and stir even, precisely test pH before filling into containers for disinfection. Keep pH between 4 and 6.9. For edible HCl aqueous solution, pH should be controlled between 6.5 and 6.9.

In industrial situation, the said HCl aqueous solution can be in mass manufactured continuously and stably by applying the pH controller apparatus.

The HCl injection formulation can be made according to common pharmaceutical method only if the pH of the injection is controlled between 3 and 6.9 (4-6.5 is preferable).

In case tap water is used to prepare HCl water, the beginning part of the production line should be equipped with a water purification apparatus ensuring the water inlet to meet national drinking-water standards.

To treat hypertension, the said HCl in the present invention can be also applied to other solutions such as physiological salt solution, glucose solution and beverages, sharing common method with the above only if pH is controlled as required.

The edible HCl water described by the present invention should not be heated for drinking because heating will evaporate HCl, so that losing therapeutic effect. Beverages containing binding materials and tea should be avoided because base materials can neutralize HCl.

To show evidence of the therapeutic effect of HCl to hypertension, the present invention made clinical observation on 126 hypertension patients who drink the HCl aqueous solution. The subjects included 87 male patients and 39 female. Average age of them was 68.2 ranging from 51-81 years old.

The results indicated that 89 patients were cured by drinking HCl water in combination with anti-hypertension medications and exclusively drinking edible HCl water; 30 patients restored normal blood pressure though halving his anti-hypertension drug dosage in combination with drinking HCl water; and 7 patients obtained normal blood pressure after having drunk edible HCl water for two years, in the past before they took the HCl water, they were unable to have a normal blood pressure although they took anti-hypertension medications the same as the time they took HCl water.

The present invention also observed patients suffering from renal, hepatic and/or biliary calculus, indicating that drinking HCl water helps alleviate or remove calculus.

Medications that are prepared from HCl and for treatment of hypertension and calculus described by the present invention are safe and reliable, and have credible and obvious effect. They can also significantly relieve hypertension patients with hardened arterial vessels, and can adapt harmful calcium salts to be calcium that are needed by human body.

### IV Detailed Description of the Embodiments

Hereinafter, the present invention will be described more specifically with reference to the following Examples. The following Examples are for illustrative purpose and are not intended to limit the scope of the invention. For Examples that have not been attached with concrete condition for the experimental methods, the acknowledged condition or condition denoted by manufacturers can be referred to. Portion or percentage refers to weight portion or weight percentage except for those that have been specially remarked.

### Example 1

Pour highly purified dilute HCl into drinkable water and stir even, precisely control pH of 6.5-7. This kind of solution can be orally taken directly.

### Example 2

Pour highly purified HCl into drinkable water to form 2% diluted HCl solution. The solution is then poured in a vessel equipped with a flow volume controller. Mix the HCl solution and drinkable water via pipes at a certain volume ratio, and at the end part of the pipe a pH detector is installed. Keeping the pH of the HCl aqueous solution in the range of 6.5 to 6.9, thus obtains edible HCl water.

The above edible HCl water is administered to 16 hypertension patients at a daily dosage of 2000ml. The patients took 500ml one hour before breakfast and lunch respectively, and 1000ml before supper.

Table 1 demonstrates alleviation of hypertension after having taken edible HCl water.

Table 1 Alleviation of hypertension after having taken edible HCl water.

**Table 1 illustrates that HCl aqueous solution is very effective in treating hypertension with a total effectiveness rate of 100%.**

| Patient number | Original blood pressure | Age | Therapeutic effect |
|---|---|---|---|
| 1 | 130/110 | 51 | The patient halved dosage one year later, and stopped using the anti-hypertension drugs in July 2002 for 2 years to date, the patient now does not use any anti-hypertension drugs, and the blood pressure is 120/80. |
| 2 | 180/110 | 75 | After the patient had been drinking edible HCl water for one and a half year, the patient halved his dosage and his blood pressure was stable. Three years later the patient stopped using anti-hypertension drugs, and the blood pressure was 110/70, the patient has abandoned anti-hypertension drugs for two years and the blood pressure keeps stable at 110/70. |
| 3 | 180/95 | 74 | The patient stopped using anti-hypertension drugs after he had been drinking HCl water for three years, and the blood pressure was 130/80. The patient has stopped using anti-hypertension drugs for two years, and the blood pressure keeps stable at 130/80. |
| 4 | 180/105 | 71 | The patient stopped using anti-hypertension drugs after he had been drinking HCl water for one and a half years, his blood pressure is 130/80. He has abandoned anti-hypertension drugs for two years and his blood pressure keeps stable at 130/80. |
| 5 | 150/90 | 75 | The patient stopped using anti-hypertension drugs after he had been drinking HCl water for three years. He has abandoned anti-hypertension drugs for two years and his blood pressure keeps stable at 130/85. |
| 6 | 200/100 | 82 | The patient halved his anti-hypertension drugs dosage after he had been drinking HCl water for one year, his blood pressure is 140/90. |
| 7 | 160/95 | 75 | The patient stopped using anti-hypertension drugs after he had been drinking HCl water for three years. He has abandoned anti-hypertension drugs for one and a half years and his blood pressure is 135/85. |
| 8 | 190/85 | 79 | The patient halved his anti-hypertension drugs after he had been drinking HCl water for three years, and his blood pressure is 130/80. |
| 9 | 150/90 | 73 | The patient stopped using anti-hypertension drugs after he had been drinking HCl water for two years. He had abandoned anti-hypertension drugs for one and a half years and his blood pressure is 133/73. |
| 10 | 160/100 | 74 | The patient stopped using anti-hypertension drugs after he had been drinking HCl water for three years. He has abandoned anti-hypertension drugs for two years and his blood pressure is 120/80. |
| 11 | 180/95 | 66 | The patient stopped using anti-hypertension drugs after he had been drinking HCl water for four years. He had abandoned anti-hypertension drugs for one year and his blood pressure is 120/80. |
| 12 | 180/110 | 72 | The patient halved his anti-hypertension drugs after he had been drinking HCl water for two years and his blood pressure is 128/65. |
| 13 | 170/110 | 62 | The patient stopped using anti-hypertension drugs after he had been drinking HCl water for three years. He has abandoned anti-hypertension drugs for one year and his blood pressure is 140/90. |
| 14 | 160/98 | 74 | The patient stopped using anti-hypertension drugs after he had been drinking HCl water for three years. He has abandoned anti-hypertension drugs for three years and his blood pressure is 135/80. |
| 15 | 160/100 | 63 | The patient stopped using anti-hypertension drugs after he had been drinking HCl water for three years. He has abandoned anti-hypertension drugs for two years and his blood pressure is 125/80. |
| 16 | 145/95 | 70 | The patient stopped using anti-hypertension drugs after he had been drinking HCl water for two years. He has abandoned anti-hypertension drugs for two years and his blood pressure is 120/75. |

### Example 3

Pour highly purified HCl into drinkable water to form 2% diluted HCl solution. The solution is then poured in a vessel equipped with a flow volume controller. Mix the HCl solution and drinkable water via pipes at a certain volume ratio, and at the end part of the pipe a pH detector is installed. Keeping the pH of the HCl aqueous solution in the range of 4.0 to 6.5, thus obtains edible HCl water.

The above edible HCl water is administered to patients suffering from calculus at a daily dosage of 2000ml given the HCl water has a pH 6.5. The patients took 500ml one hour before breakfast and lunch respectively, and 1000ml before supper. In case pH of the HCl water is lower than 6.5, volume of water can be reduced. For instance, given the HCl water has a pH 5.0, the daily dosage can be dwindled to 1000ml, and the patients took 250ml one hour before breakfast and lunch respectively, and 500ml before supper.

Table 2 and 3 demonstrate alleviation of calculus after having taken edible HCl water.

Table 2 Alleviation of renal calculus after having taken edible HCl water.

| Name | Diameter of calculus in kidney (cm) | Age | Therapeutic effect |
|---|---|---|---|
| Zhang | 0.9 | 77 | The diameter turned to be 0.48 after the patient had been drinking HCl water for one year and the calculus disappeared two years later |
| Ju | 0.41 | 70 | The calculus disappeared after the patient had been drinking HCl water for one year. |
| Mei | 0.9 in left kidney, and 0.8 in right kidney | 29 | The diameter turned to be 0.5 (left kidney) and 0.45 (right kidney) after the patient had been drinking HCl water for one year, and the calculus disappeared two years later. |
| Lin | 0.52 | 46 | The calculus disappeared after the patient had been drinking HCl water for one year. |
| Han | 0.8 | 63 | The diameter turned to be 0.5 after the patient had been drinking HCl water for one year and the calculus disappeared one and a half years later. |
| Zhou | 1.2 | 52 | The diameter turned to be 0.8 after the patient had been drinking HCL water for one year and disappeared two years later. |
| Huang | 0.95 | 38 | The diameter turned to be 0.52 after the patient had been drinking HCL water for one year and the calculus disappeared two years later |
| Gao | 0.6 | 45 | The calculus disappeared after the patient had been drinking HCl water for one year. |
| Zhang | 0.7 | 21 | The calculus disappeared after the patient had been drinking HCl water for ten months. |
| Cao | 0.65 | 62 | The diameter turned to be 0.5 after the patient had been drinking HCl water for a half year and the calculus disappeared one and a half years later. |

**Table 3 Alleviation of hepatic and biliary calculus after having taken edible HCl water.**

| Name | Diameter of calculus in liver and gallbladder (cm) | Age | Therapeutic effect |
|---|---|---|---|
| Tan | 0.7x0.6 | 63 | The calculus was cured after the patient had been drinking HCl water for ten months (confirmed by B-type ultrasound inspection). |
| Ying | 0.7 | 53 | The diameter turned to be 0.4 after the patient had been drinking HCl water for one year, and the calculus disappeared one and a half year later. |
| Xu | 0.8 | 74 | The calculus disappeared after the patient had been drinking HCl water for two years. |
| Zhang | 1.6 | 69 | The diameter turned to be 1.2 after the patient had been drinking HCl water for one year, and turned to be 0.6 after the patient had been drinking HCl water for two years, the calculus disappeared two and a half years later. |
| Lu | Two stones have a diameter of 1.5 and one stone has a diameter of 0.8 | 75 | The diameter of two stones turned to be 0.7 after the patient had been drinking HCl water for one year, and the calculus disappeared two and a half years later. |
| Chen | Φ1.3 | 52 | The diameter turned to be 0.8 after the patient had been drinking HCL water for one year (confirmed by B-type ultrasound inspection). |
| Liu | Φ1.8 | 65 | The diameter turned to be 1.1 after the patient had been drinking HCL water for one year and turned to be 0.6 after the patient had been drinking HCL water for two years. |
| Liu | Φ1.1 | 62 | The calculus disappeared after the patient had been drinking HCl water for one and a half years. |
| Ni | Φ1.9×5 | 55 | The diameter turned to be 1.6x4.1 after the patient had been drinking HCL, water for one year and turned to be 1.6x3.1 after the patient had been drinking HCL water for two years. |
| Zhu | Φ2.1 | 67 | The diameter turned to be 1.1 after the patient had been drinking HCl water for one and a half years. |

Table 2 and 3 illustrate that HCl water is significantly effective in treating renal, hepatic and biliary calculus.

### Example 4

A compound formulation consisting of HCl aqueous solution and anti-hypertension drugs.

Add one tablet (2.5mg) of market available Indapamide into 500ml of HCl aqueous solution of pH 6.9, making up a compound formulation that contains HCl and Indapamide.

Similarly, other compound formulations that contain HCl and anti-hypertension drugs can be obtained.

All the references mentioned in this specification are herein incorporated into the specification, to the same extent as if each individual reference was specifically and individually indicated to be incorporated herein by reference. It should be understood that after having reviewed the above contents of the present invention, those ordinary skilled of the art can make variations and modifications to the present invention, which are still within the spirit and scope of the appended claims.

## Claims

1. The use of HCl for the manufacture of medicament for the treatment of hypertension.

2. The use of HCl for the manufacture of medicament for the treatment of hypertension as in claim 1, wherein, the anti-hypertension medicament is an aqueous solution that contains HCl, and the said aqueous solution has a pH of 3-6.9.

3. The use of HCl for the manufacture of medicament for the treatment of hypertension as in claim 2, wherein, the said aqueous solution has a pH of 6.5-6.9.

4. The use of HCl for the manufacture of medicament for the treatment of hypertension as in claim 2 or 3, wherein, the said anti-hypertension medicament is in the form of oral formulation.

5. The use of HCl for the manufacture of medicament for the treatment of hypertension as in claim 2, wherein, the said anti-hypertension medicament is in the form of injection formulation.

6. The use of HCl for the manufacture of medicament for the treatment of hypertension as in claim 1, wherein, the said anti-hypertension medicament is a HCl aqueous solution that has a pH 3-6.9, and contains other anti-hypertension medications that can keep stable within the above mentioned pH range.

7. A method for treating hypertension, wherein, the subject was administered the HCl aqueous solution of pH 3-6.9 of 100-300ml each day for 0.5-6 years.

8. An anti-hypertension formulation, wherein, the formulation is a HCl aqueous solution that has a pH of 3-6.9, and contains anti-hypertension medications that can keep stable within above mentioned pH ranges and are selected from the group consisting of Amlodipine, Benazepril, Captopril, Clonidine, Enalaprilat, Felodipin, Guanazodine, Indanidine, Indapamide, Minoxidil, Nitrendipine, Perindopril, Proroxan, Reserpiline, Trimoxamine, Tripamide, Utibapril, Zabiciprilat or their combinations.

9. The use of HCl for the manufacture of medicament for the treatment of calculus.

10. The use of HCl for the manufacture of medicament for the treatment of calculus as in claim 9 wherein the said calculus diseases are selected from the group consisting of renal, hepatic or biliary calculus.
